# EUROPEAN PATENT APPLICATION

(11) **EP 2 296 252 A1**
(43) Date of publication of application: **16.03.2011**
(21) Application number: 09734885.8
(22) Date of filing: 21.04.2009
(51) Int. Cl.: H02J 17/00

(54) **POWER TRANSMISSION SYSTEM**

(30) Priority: 22.04.2008 JP 2008111608
(71) Applicant: Olympus Corporation, Shibuya-ku Tokyo 151-0072 (JP)
(72) Inventor: SATO, Ken, Tokyo 151-0072 (JP)
(74) Representative: Winter, Brandl, Fürniss, Hübner, Röss, Kaiser, Polte - Partnerschaft
(86) International application number: PCT/JP2009/057924
(87) International publication number: WO 2009/131121

(57) **Abstract**

A power transmission system for supplying energy to a device operating on electrical energy taken by a power receiving antenna includes a driving unit supplied with electric power from a power supply and generating an AC current and a power transmission antenna receiving the AC current from the driving unit and generating an electromagnetic field. The power transmission antenna includes a resonance frequency adjusting circuit adjusting and setting a resonance frequency.

## Description

### Technical Field

The present invention relates to a power transmission system which wirelessly supplies electric power from outside of a body to a small medical device operating inside of the body.

### Background Art

Energy supply apparatuses that contactlessly supply electrical energy to a certain device have been proposed such as the one disclosed in Japanese Patent Application Laid-Open Publication No. 2004-159456 in which an electric current is passed through a primary coil provided in the energy supply apparatus to induce electrical energy in a secondary coil provided in the device.

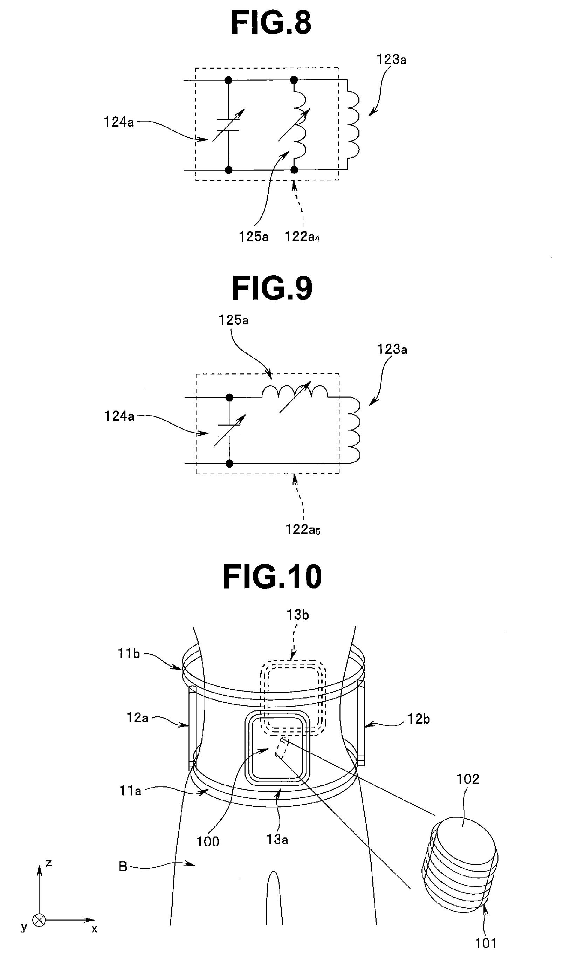

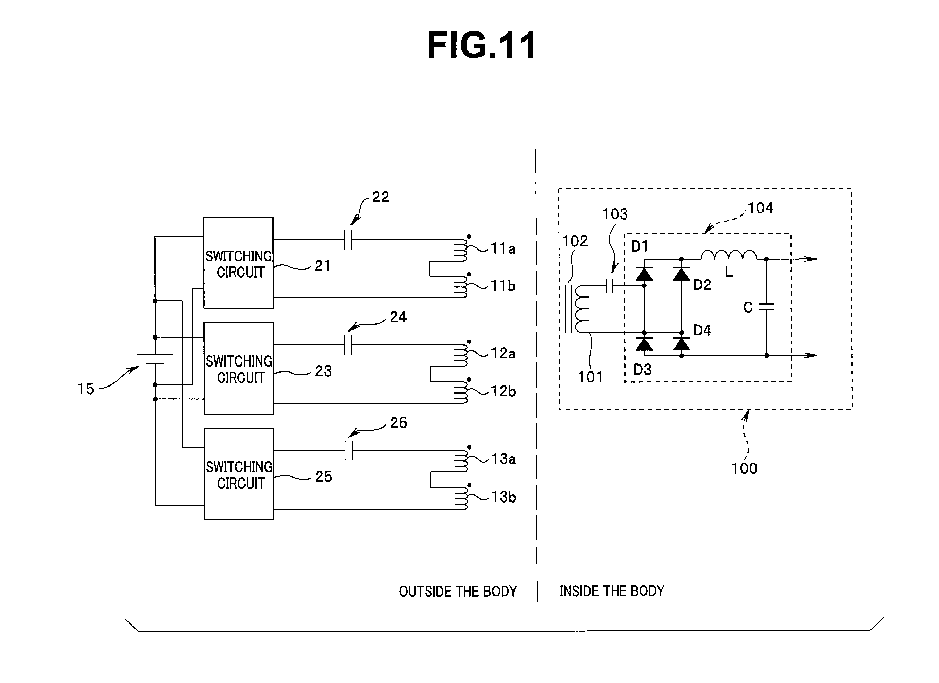

The configuration of the primary coil provided in the energy supply apparatus in the proposal described in the Japanese Patent Application Laid-Open Publication No. 2004-159456 will be briefly described below with reference to Figs. 10 and 11.

Fig. 10 illustrates a primary coil configuration of an existing energy supply apparatus. Illustrated in Fig. 10 is the configuration of a wireless power supply system for capsule endoscope in which X-, Y-, and Z-axis primary coils are attached to the body of a subject B and electric power is wirelessly supplied to the capsule endoscope, which is a small medical device, in a body cavity of the subject B.

In Fig. 10, the primary coils are arranged on the body of the subject B along the X-, Y-, and Z-axes that are orthogonal to each other. Primary coils 12a and 12b are located along the X-axis; primary coils 13a and 13b are located along the Y-axis; primary coils 11a and 11b are located along the Z-axis. The capsule endoscope 100 is located in a body cavity of the subject B and a secondary coil 101 is contained in the capsule endoscope 100. Electric power required for causing the capsule endoscope 100 to operate is induced and supplied in the secondary coil 101 by interlinkage of electromagnetic induction phenomenon by magnetic fields generated by the primary coils 11 to 13 with the secondary coil 101 contained in the capsule endoscope 100.

Fig. 11 illustrates a circuit configuration of the primary coils in the existing energy supply apparatus. When electrical energy is supplied to the capsule endoscope 100, the multiple primary coils 11a and 11b, 12a and 12b, and 13a and 13b, respectively, are connected in series as illustrated in Fig. 11. The pairs of primary coils 11a and 11b, 12a and 12b, and 13a and 13b connected in series are connected to switching circuits 21, 23 and 25, respectively, which are primary coil driving circuits, through primary coil resonant capacitors 22, 24 and 26, respectively.

A driving DC power supply 15 is connected to the switching circuits 21, 23 and 25. When high-frequency voltages outputted from the switching circuits 21, 23 and 25 are applied to the circuit in which the multiple primary coils and the resonant capacitors are connected in series, the primary coils 11a and 11b forms a series resonance circuit with the capacitor 22, the primary coils 12a and 12b forms a series resonance circuit with the capacitor 24, and the primary coils 13a and 13b forms a series resonance circuit with the capacitor 26, thereby generating a magnetic field in the direction of the axis of each primary coil.

By driving the primary coils 11a, 11b, 12a, 12b, 13a, and 13b in this way, electrical energy can be supplied to the capsule endoscope 100.

The proposal in Japanese Patent Application Laid-Open Publication No. 2004-159456 describes only a technique in which only one energy supply apparatus is used. No mention is made of a technique in which multiple energy supply apparatuses are located close to each other and operated. In a medical practice, there can be a situation in which separate energy supply apparatuses are used to cause small medical devices in body cavities of multiple subjects located close to each other to operate and medical checks or examinations are conducted. If multiple existing energy supply apparatuses described in Japanese Patent Application Laid-Open Publication No. 2004-159456 are located close to each other in a situation as described above, power transmission antennas provided in the energy supply apparatuses can magnetically couple with each other to interfere with each other.

When multiple power transmission antennas provided in different energy supply apparatuses magnetically couple with each other, mutual induction causes induced current in the power transmission antennas. In particular, if power transmission antennas, each including a resonance circuit, are used to supply energy to small medical devices in body cavities and resonance frequencies of the multiple energy supply apparatuses located close to each other are the same, a large current is induced in each power transmission antenna.

In the proposal described in Japanese Patent Application Laid-Open Publication No. 2004-159456, no mention is made of operation when the resonance frequencies of power transmission antennas provided in multiple energy supply apparatuses located in close to each other are the same. Thus, the resonance frequencies of multiple existing energy supply apparatuses located close to each other can be equal to each other.

If the resonance frequencies of closely located energy supply apparatuses are equal and a current induced in each power transmission antenna increases, control of the current flowing through the power transmission antenna becomes difficult, energy supply to the small medical device operating inside the body becomes unstable, and operation of the small medical device becomes disadvantageously unstable.

The present invention has been made in light of these circumstances and an object of the present invention is to provide a power transmission system in which multiple energy supply apparatuses that wirelessly supply power from the outside of bodies are capable of stably supplying energy even when the energy supply apparatuses are located close to each other.

### Disclosure of Invention

### Means for Solving the Problem

According to one embodiment of the present invention, a power transmission system for supplying energy to a device operating on electrical energy taken by a power receiving antenna includes a driving unit supplied with electric power from a power supply and generating an AC current, a power transmission antenna receiving the AC current from the driving unit and generating an electromagnetic field to supply the electrical energy to the device, and a resonance frequency adjusting circuit provided in the power transmission antenna and adjusting and setting a resonance frequency.

### Brief Description of the Drawings

Fig. 1 is a schematic diagram illustrating a configuration of energy supply apparatuses 1a and 1b according to a first embodiment of the present invention;
Fig. 2 is a circuit diagram illustrating an exemplary circuit configuration of a resonance frequency adjusting circuit 122a;
Fig. 3 is a diagram illustrating exemplary frequency characteristics of the energy supply apparatuses 1a and 1b;
Fig. 4 is a diagram illustrating exemplary frequency characteristics of power receiving antennas 201a and 201b;
Fig. 5 is a circuit diagram illustrating a variation of the circuit configuration of a resonance frequency adjusting circuit 122a₁;
Fig. 6 is a circuit diagram illustrating a variation of the circuit configuration of a resonance frequency adjusting circuit 122a₂;
Fig. 7 is a circuit diagram illustrating a variation of the circuit configuration of a resonance frequency adjusting circuit 122a₃;
Fig. 8 is a circuit diagram illustrating a variation of the circuit configuration of a resonance frequency adjusting circuit 122a₄;
Fig. 9 is a circuit diagram illustrating a variation of the circuit configuration of a resonance frequency adjusting circuit 122a₅;
Fig. 10 is a diagram illustrating a primary coil configuration in an existing energy supply apparatus; and
Fig. 11 is a circuit configuration diagram of primary coils in the existing energy supply apparatus.

### Best Mode for Carrying Out the Invention

Embodiments of the present invention will be described below with reference to drawings.

### (First embodiment)

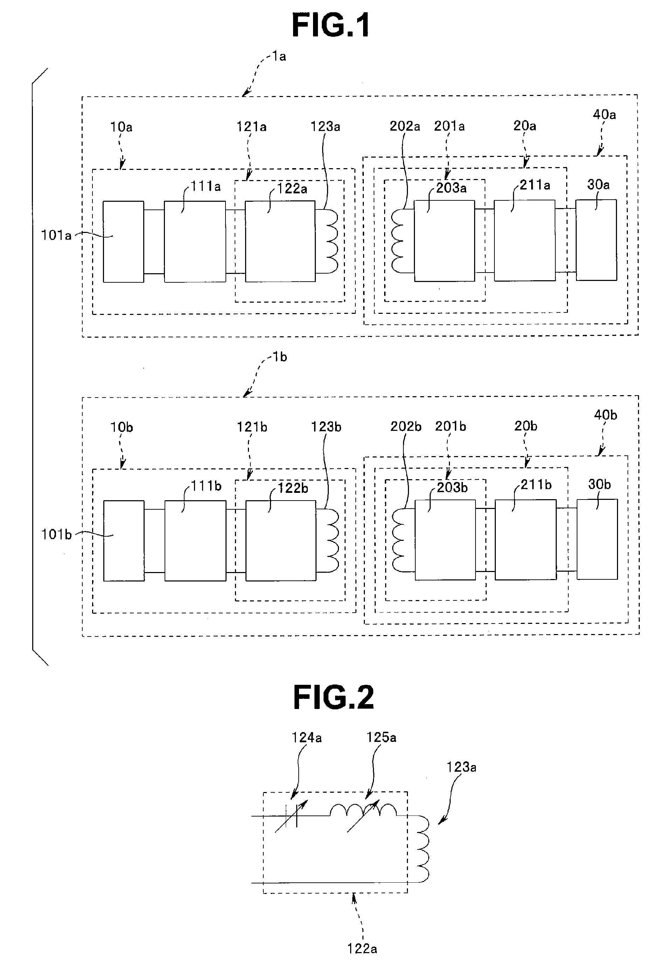

A configuration of an energy supply apparatus will be described first with reference to Fig. 1. Fig. 1 is a schematic diagram illustrating a configuration of energy supply apparatuses 1a and 1b according to a first embodiment of the present invention. In Fig. 1, two energy supply apparatuses 1a and 1b are located close to each other.

The energy supply apparatuses 1a and 1b have the same configuration and therefore only the configuration of the energy supply apparatus 1a will be described in detail herein and description of the configuration of the energy supply apparatus 1b will be omitted.

In Fig. 1, the energy supply apparatus 1a includes a small medical device 40a including a power receiving system 20a for receiving electrical energy and a device 30a, and a power transmission system 10a supplying electrical energy to the small medical device 40a.

The power transmission system 10a is configured to generate an electromagnetic field and supply electric power to the power receiving system 20a and includes a power supply 101a, a driving unit 111a, and power transmission antenna 121a.

The driving unit 111a is supplied with electric power from the power supply 101a and applies an AC current to the power transmission antenna 121a. The driving unit 111a includes a current sensor, not depicted, which measures a current flowing through the power transmission antenna 121a.

The power transmission antenna 121a includes a resonance frequency adjusting circuit 122a and a power transmission coil 123a. The resonance frequency adjusting circuit 122a is a reactance adjusting circuit and is functionally configured to be capable of resonating with the power transmission coil 123a at a given frequency f_{A}. When an AC current is applied to the power transmission antenna 121a thus configured, an electromagnetic field is generated.

The power receiving system 20a includes a power receiving antenna 201a and a power receiving circuit 211a. The power receiving antenna 201a includes a power receiving coil 202a and a resonance circuit 203a. The resonance circuit 203a includes a capacitor.

In the power receiving antenna 201a, electrical energy is taken from an electromagnetic field generated at the power transmission antenna 121a. The taken electrical energy is transmitted to the power receiving circuit 211a, where the electrical energy is converted to a form of power appropriate for operation of the device 30a. The device 30a is a main functional unit of the small medical device 40a. For example, if the small medical device 40a is a capsule endoscope, the device 30a includes components such as an image pickup unit, an image processing unit, and an information communication unit. These components operate on electric power provided from the power receiving system 20a.

A resonance frequency adjusting operation in the energy supply apparatus 1a, 1b configured as described above will be described.

A resonance frequency f_{A} of the power transmission antenna 121a is set by the resonance frequency adjusting circuit 122a. Similarly, a resonance frequency f_{B} of the power transmission antenna 121b is set by the resonance frequency adjusting circuit 122b.

Here, if frequencies f_{A} and f_{B} are set to the same or approximately the same value, induced currents flowing through the power transmission antennas 121a and 121b increase and energy supply to the small medical devices 40a and 40b become unstable when the energy supply apparatuses 1a and 1b are located close to each other.

Therefore, in order to reduce induced currents flowing through the power transmission antennas 121a and 121b, resonance frequency f_{A} or f_{B} is adjusted and set by the resonance frequency adjusting circuit 122a or 122b so that resonance frequency f_{A} of the power transmission antenna 121a and resonance frequency f_{B} of the power transmission antenna 121b differ from each other.

A circuit configuration of the resonance frequency adjusting circuits 122a and 122b will be described below with reference to Fig. 2. Fig. 2 is a circuit diagram illustrating an exemplary circuit configuration of the resonance frequency adjusting circuit 122a. As has been stated above, the resonance frequency adjusting circuit 122a has a circuit configuration in which reactance can be adjusted, for example, a circuit configuration in which a capacitor 124a and an inductor 125a are connected in series as illustrated in Fig. 2.

The value of at least one of capacitance of the capacitor 124a and inductance of the inductor 125a is variable. By adjusting the value, the reactance of the entire circuit can be adjusted.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

On the other hand, circuit configurations of the inductor 125a that allow the inductance of the entire inductor 125a to be adjusted include, in addition to a single variable inductor, a group of variable inductors in which multiple variable inductors are connected, a group of inductors in which a fixed inductor(s) and a variable inductor(s) are provided in a mixed manner and connected, a group of switching inductors in which fixed inductors are connected with a switch and the switch is turned on and off to change the inductance of the entire inductor circuit, a switch-tapped inductor in which an inductor has multiple taps and switching is made between the taps by using switches to change the inductance, and various other circuit configurations.

By changing the reactance of the resonance frequency adjusting circuit 122a configured as described above, the value of resonance frequency f_{A} can be adjusted and set. The resonance frequency adjusting circuit 122b has the same circuit configuration as the resonance frequency adjusting circuit 122a described with reference to Fig. 2 and therefore detailed description of the resonance frequency adjusting circuit 122b will be omitted. Reactance of the resonance frequency adjusting circuit 122b therefore can be changed to adjust and set the value of resonance frequency f_{B}.

The circuit configuration of the resonance frequency adjusting circuits 122a and 122b is not limited to those described above. Any of various other circuit configurations can be used without departing from the spirit of the present invention. Examples of other circuit configurations will be detailed with respect to other embodiments which will be described later.

The resonance frequency of one of the power transmission systems is set to a value such that the magnitude of a current induced in the power transmission antenna provided in the other power transmission system when a current is applied to the power transmission antenna provided in the power transmission system will be small. Accordingly, the resonance frequency of one of the power transmission systems is set to a value different from the value of the resonance frequency of the other power transmission system.

For example, in the case of the energy supply apparatuses 1a and 1b described above, resonance frequency f_{A} of the power transmission antenna 121a is set to a value such that a current induced in the power transmission antenna 121b when a current is applied to the power transmission antenna 121a will be small. That is, resonance frequency f_{A} is set to a value different form that of resonance frequency f_{B}. Resonance frequencies f_{A} and f_{B} are manually or automatically adjusted and set when the energy supply apparatuses 1a and 1b are installed or activated.

The driving unit 111a is supplied with electric power from the power supply 101a and applies an AC current that is approximately equivalent to resonance frequency f_{A} of the power transmission antenna 121a set by the resonance frequency adjusting circuit 122a to the power transmission antenna 121a. Similarly, the driving unit 111b is supplied with electric power from the power supply 101b and applies an AC current that is approximately equivalent to resonance frequency f_{B} of the power transmission antenna 121b set by the resonance frequency adjusting circuit 122b to the power transmission antenna 121b.

In the power receiving antennas 201a and 201b which takes electrical energy from electromagnetic fields generated by the power transmission antennas 121a and 121b, the resonance circuits 203a and 203b, respectively, set resonance frequencies. There are three possible principal methods for setting the resonance frequencies in the power receiving antennas 201a and 201b. One of the methods will be described with respect to the present embodiment and the other methods will be detailed later with respect to other embodiments which will be described later.

The resonance frequencies of the power receiving antennas 201a and 202b in the present embodiment have been set to values specific to the respective antennas at the time of manufacturing of the small medical devices 40a and 40b. That is, when the energy supply apparatus is used, a power transmission system and a small medical device equipped with a power receiving antenna in which a resonance frequency that is approximately equal to the resonance frequency of the power transmission antenna of the power transmission system are selected and used in combination.

In the configuration illustrated in Fig. 1, when the energy supply apparatus 1a is used, the small medical device 40a equipped with the power receiving antenna 201a in which a resonance frequency approximately equal to resonance frequency f_{A} of the power transmission antenna 121a is set is selected and used in combination with the power transmission system 10a. When the energy supply apparatus 1b is used, the small medical device 40b equipped with the power receiving antenna 201b in which a resonance frequency approximately equal to resonance frequency f_{B} of the power transmission antenna 121b is selected and used in combination with the power transmission system 10b.

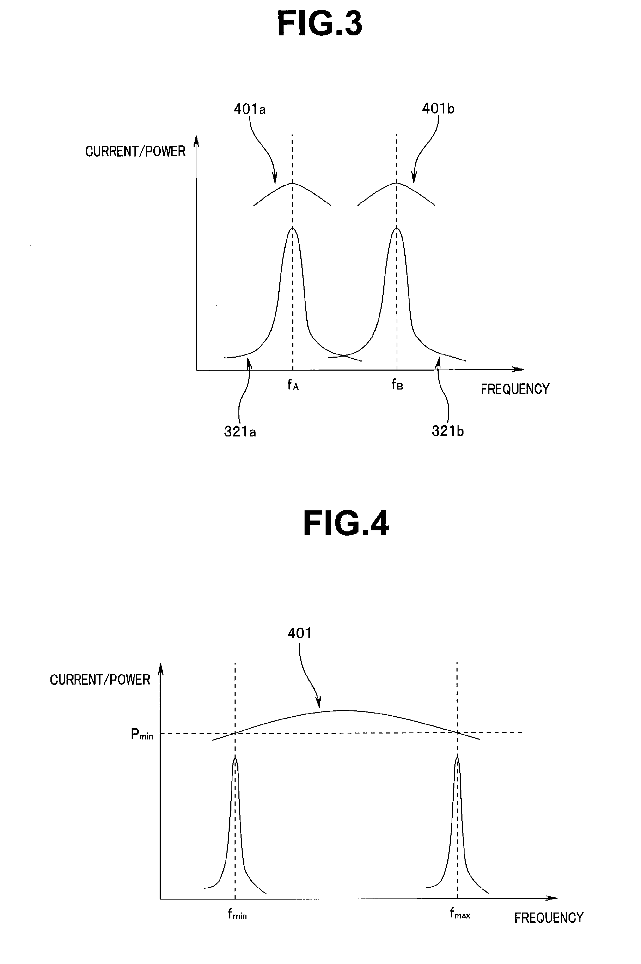

Fig. 3 illustrates exemplary frequency characteristics of the energy supply apparatuses 1a and 1b configured as described above. In Fig. 3, the horizontal axis represents frequency and the vertical axis represents current flowing through the power transmission antennas 121a and 121b and electric power received at the power receiving antennas 201a and 201b. In Fig. 3, the frequency characteristic of the power transmission antenna 121a is indicated by 321a, the frequency characteristic of the power transmission antenna 121b is indicated by 321b, the frequency characteristic of the power receiving antenna 201a is indicated by 401a, and the frequency characteristic of the power receiving antenna 201b is indicated by 401b.

As illustrated in Fig. 3, the resonance frequency of the power transmission antenna 121a is f_{A} and the resonance frequency of the power transmission antenna 121b is f_{B} (≠ f_{A}). On the other hand, the resonance frequency of the power receiving antenna 201a is f_{A}, which is equal to the resonance frequency of the power transmission antenna 121a, and the resonance frequency of the power receiving antenna 201b is f_{B}, which is equal to the resonance frequency of the power transmission antenna 121b.

That is, the resonance frequency f_{A} of the power transmission antenna 121a contained in one 1a of the two energy supply apparatuses 1a and 1b located close to each other is set to a value different from the resonance frequency f_{B} of the power transmission antenna 121b contained in the other energy supply apparatus 1b in the present embodiment. The small medical device 40a equipped with the power receiving antenna 201a having a resonance frequency approximately equal to resonance frequency f_{A} of the power transmission antenna 121a is used in combination with the power transmission system 10a; the small medical device 40b equipped with the power receiving antenna 201b having a resonance frequency approximately equal to resonance frequency f_{B} of the power transmission antenna 121b is used in combination with the power transmission system 10b.

By configuring the energy supply apparatuses 1a and 1b in this way, electric power from an electromagnetic field generated at the power transmission antenna 121a can be reliably received at the power receiving antenna 201a, electric power from an electromagnetic field generated at the power transmission antenna 121b can be reliably received at the power receiving antenna 201b, and an induced current caused by interference between the power transmission antennas 121a and 121b can be minimized. Accordingly, each of the apparatuses can supply energy stably and reliably.

While a situation has been described in which two energy supply apparatuses 1a and 1b are located close to each other in the present embodiment, it will be understood that the same effect can be provided in a situation where more than two energy supply apparatuses are located close to each other with a configuration and resonance frequency settings similar to those described above.

### (Second embodiment)

An energy supply apparatus according to a second embodiment of the present invention will be described below in detail.

The energy supply apparatus in the present embodiment has the same configuration as the energy supply apparatus of the first embodiment described with reference to Fig. 1, including the circuit configuration of resonance frequency adjusting circuits 122a and 122b, except the method in which resonance frequencies are set in power receiving antennas 201a and 201b, in particular the configuration of resonance circuits 203a and 203b contained in power receiving systems 20a and 20b. Therefore only the configuration of resonance circuits 203a and 203b will be described here and the same components as those of the first embodiment will be given the same reference symbols and description of the same components will be omitted.

In the first embodiment, the resonance frequencies of the power receiving antennas 201a and 201b are set to values specific to the power receiving antennas 201a and 201b, at the time of manufacturing of the small medical devices 40a and 40b. In contrast, resonance frequencies of the power receiving antennas 201a and 201b in the present embodiment are variable.

Specifically, the resonance circuits 203a and 203b of the energy supply apparatuses in the present embodiment include a capacitor, not depicted, the capacitance of which is adjustable. The capacitor is connected with power receiving antenna 201a, 201b in parallel or in series.

When the energy supply apparatuses are used, the capacitance of the capacitor of the resonance circuit 203a is adjusted so that the resonance frequency of the power receiving antenna 201a becomes approximately equal to the resonance frequency of the power transmission antenna 121a and the capacitance of the capacitor of the resonance circuit 203b is adjusted so that the resonance frequency of the power receiving antenna 201b becomes approximately equal to the resonance frequency of the power transmission antenna 121b. Thus, the resonance frequencies are set so as to exhibit frequency characteristics as illustrated in Fig. 3.

Since the resonance frequencies of the power receiving antennas 201a and 201b can be adjusted at the time of use in the present embodiment as described above, internal configurations of small medical devices 40a and 40b can be made identical irrespective of the resonance frequencies of the power transmission antennas 121a and 121b. Accordingly, the energy supply apparatuses 1a and 1b can use small medical devices with the same configuration and specifications and therefore manufacturing costs of the small medical devices can be reduced. Furthermore, since there is no need for providing power receiving systems 20a and 20b of different resonance frequencies and no need for selecting power receiving systems 20a, 20b that are compatible with the resonance frequencies of power transmission systems 10a, 10b at the time of use, convenience to use is improved.

### (Third embodiment)

An energy supply apparatus according to a third embodiment of the present invention will be described below in detail.

The energy supply apparatus in the present embodiment has the same configuration as the energy supply apparatus of the first embodiment described with reference to Fig. 1, including the circuit configuration of resonance frequency adjusting circuits 122a and 122b, except the method in which resonance frequencies are set in power receiving antennas 201a and 201b, in particular the configuration of resonance circuits 203a and 203b contained in power receiving systems 20a and 20b. Therefore only the configuration of resonance circuits 203a and 203b will be described here and the same components as those of the first embodiment will be given the same reference symbols and description of the same components will be omitted.

In the first embodiment, the resonance frequencies of the power receiving antennas 201a and 201b have been set to values specific to the power receiving antennas 201a and 201b at the time of manufacturing of the small medical devices 40a and 40b and the power receiving antennas 201a and 201b with resonance frequencies approximately equal to the resonance frequencies of the power transmission antennas 121a and 121b are selected and used. While the resonance frequencies of power receiving antennas 201a and 201b in the third embodiment also have been set to values specific to the power receiving antennas 201a and 201b at the time of manufacturing, small medical devices 40a and 40b in the third embodiment, unlike those in the first embodiment, are equipped with the power receiving antennas 201a and 201b having the same resonance frequency characteristics regardless of the resonance frequencies of power transmission antennas 121a and 121b.

Fig. 4 illustrates exemplary frequency characteristics of the power receiving antennas 201a and 201b in the present embodiment. In Fig. 4, the horizontal axis represents frequency and the vertical axis represents current flowing through the power transmission antennas 121a and 121b and electric power received at the power receiving antennas 201a and 201b. In Fig. 4, a minimum resonance frequency of the power transmission antennas that can be adjusted and set is indicated by fₘᵢₙ, a maximum resonance frequency of the power transmission antennas that can be adjusted and set is indicated by fₘₐₓ, and a minimum received electric power required for power receiving systems of small medical devices is indicated by Pₘᵢₙ. Frequency characteristics of the power receiving antennas 201a and 201b are indicated by 401.

As illustrated in Fig. 4, the frequency characteristics of the power receiving antennas 201a and 201b are set so that the power receiving antennas 201a and 201b can receive electric power greater than or equal to the electric power Pₘᵢₙ required for power receiving systems, provided that the resonance frequencies of the power transmission antennas 121a, 121b are in the range between fₘᵢₙ and fₘₐₓ.

In the present embodiment as described above, the internal configurations of small medical devices 40a and 40b can be made identical irrespective of the resonance frequency of the power transmission antennas 121a and 121b. Furthermore, since the capacitance of the capacitor of the resonance circuit 203b can be fixed, a simple configuration can be used compared with a configuration in which the capacitance can be adjustable. Accordingly, the manufacturing costs of small medical devices can be further reduced.

Moreover, since there is no need for selecting power receiving systems 20a, 20b that are compatible with the resonance frequencies of power transmission systems 10a, 10b or for adjusting the resonance frequencies of the power receiving systems 20a and 20b depending on the power transmission systems 10a and 10b to be used in conjunction at the time of use, convenience to use is further improved.

### (Fourth embodiment)

An energy supply apparatus according to a fourth embodiment of the present invention will be described below in detail.

The energy supply apparatus in the present embodiment has the same configuration as the energy supply apparatus of the first embodiment described with reference to Fig. 1, including the method in which the resonance frequencies are set in power receiving antennas 201a and 201b, in particular the configuration of resonance circuits 203a and 203b contained in power receiving systems 20a and 20b, except the circuit configuration of resonance frequency adjusting circuits 122a₁ and 122b₁. Therefore only the circuit configuration of the resonance frequency adjusting circuits 122a₁ and 122b₁ will be described here and the same components as those of the first embodiment will be given the same reference symbols and description of the same components will be omitted.

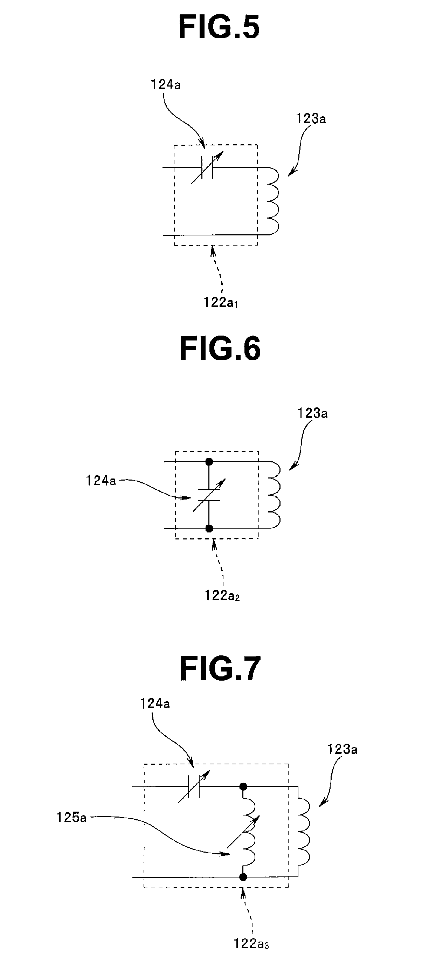

A configuration of the resonance frequency adjusting circuits 122a₁ and 122b₁ in the present embodiment will be described with reference to Fig. 5. Fig. 5 is a circuit diagram illustrating a variation of the circuit configuration of the resonance frequency adjusting circuit 122a₁. The resonance frequency adjusting circuit 122a₁ includes a capacitor 124a for adjusting and setting reactance. The capacitor 124a is connected to a power transmission coil 123a in series.

The value of capacitance of the capacitor 124a is variable. By adjusting the value, reactance of the entire circuit can be adjusted. By changing the reactance of the resonance frequency adjusting circuit 122a₁, the value of resonance frequency f_{A} can be adjusted and set. The resonance frequency adjusting circuit 122b₁ has the same circuit configuration as the resonance frequency adjusting circuit 122a₁ described with reference to Fig. 5 and therefore detailed description of the resonance frequency adjusting circuit 122b₁ will be omitted. Reactance of the resonance frequency adjusting circuit 122b₁ therefore can be changed to adjust and set the value of resonance frequency f_{B}.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

As has been described, in the present embodiment, each of the resonance frequency adjusting circuits 122a₁ and 122b₁ includes only a capacitor 124a for adjusting and setting reactance and does not need an inductor. Accordingly, the number of components of the resonance frequency adjusting circuits 122a₁ and 122a₁ can be reduced and the circuit configuration can be simplified. Consequently, the manufacturing costs of the resonance frequency adjusting circuits 122a₁ and 122b₁ can be reduced and hence the manufacturing costs of the entire energy supply apparatus can be reduced.

### (Fifth embodiment)

An energy supply apparatus according to a fifth embodiment of the present invention will be described below in detail.

The energy supply apparatus of the present embodiment has the same configuration as the energy supply apparatus of the first embodiment described with reference to Fig. 1, including the method in which resonance frequencies are set in power receiving antennas 201a and 201b, in particular the configuration of resonance circuits 203a and 203b contained in power receiving systems 20a and 20b, except the circuit configuration of resonance frequency adjusting circuits 122a₂ and 122b₂. Therefore only the circuit configuration of the resonance frequency adjusting circuits 122a₂ and 122b₂ will be described here and the same components as those of the first embodiment will be given the same reference symbols and description of the same components will be omitted.

A configuration of the resonance frequency adjusting circuits 122a₂ and 122b₂ of the present embodiment will be described with reference to Fig. 6. Fig. 6 is a circuit diagram illustrating a variation of the circuit configuration of the resonance frequency adjusting circuit 122a₂. The resonance frequency adjusting circuit 122a₂ includes a capacitor 124a for adjusting and setting reactance. While the capacitor 124a in the first and fourth embodiments is connected to a power transmission coil 123a in series, the capacitor 124a in the present embodiment is connected to the power transmission coil 123a in parallel.

The value of capacitance of the capacitor 124a is variable. By adjusting the value, reactance of the entire circuit can be adjusted. By changing the reactance of the resonance frequency adjusting circuit 122a₁, the value of resonance frequency f_{A} can be adjusted and set. The resonance frequency adjusting circuit 122b₂ has the same circuit configuration as the resonance frequency adjusting circuit 122a₂ described with reference to Fig. 6 and therefore detailed description of the resonance frequency adjusting circuit 122b₂ will be omitted. Reactance of the resonance frequency adjusting circuit 122b₂ therefore can be changed to adjust and set a value of resonance frequency f_{B}.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

As has been described, in the present embodiment, each of the resonance frequency adjusting circuits 122a₂ and 122b₂ includes only a capacitor 124a for adjusting and setting reactance and does not need an inductor. Accordingly, the number of components of the resonance frequency adjusting circuits 122a₂ and 122b₂ can be reduced and the circuit configuration can be simplified. Consequently, the manufacturing costs of the resonance frequency adjusting circuits 122a₂ and 122b₂ can be reduced and hence the manufacturing costs of the entire energy supply apparatus can be reduced.

### (Sixth embodiment)

An energy supply apparatus according to a sixth embodiment of the present invention will be described below in detail.

The energy supply apparatus of the present embodiment has the same configuration as the energy supply apparatus of the first embodiment described with reference to Fig. 1, including the method in which resonance frequencies are set in power receiving antennas 201a and 201b, in particular the configuration of resonance circuits 203a and 203b contained in power receiving systems 20a and 20b, except the circuit configuration of resonance frequency adjusting circuits 122a₃ and 122b₃. Therefore only the circuit configuration of the resonance frequency adjusting circuits 122a₃ and 122b₃ will be described here and the same components as those of the first embodiment will be given the same reference symbols and description of the same components will be omitted.

A configuration of the resonance frequency adjusting circuits 122a₃ and 122b₃ of the present embodiment will be described with reference to Fig. 7. Fig. 7 is a circuit diagram illustrating a variation of the circuit configuration of the resonance frequency adjusting circuit 122a₃. The resonance frequency adjusting circuit 122a₃ includes a capacitor 124a and an inductor 125a for adjusting and setting reactance. While both of the capacitor 124a and the inductor 125a in the first embodiment are connected to a power transmission coil 123a in series, the inductor 125a in the present embodiment is connected to the power transmission coil 123a in parallel.

The value of capacitance of the capacitor 124a and the value of inductance of the inductor 125a are variable and reactance of the entire circuit can be adjusted by adjusting these values. The value of resonance frequency f_{A} can be adjusted and set by changing reactance of the resonance frequency adjusting circuit 122a₃. The resonance frequency adjusting circuit 122b₃ has the same circuit configuration as the resonance frequency adjusting circuit 122a₃ described with reference to Fig. 7 and therefore detailed description of the resonance frequency adjusting circuit 122b₃ will be omitted. Reactance of the resonance frequency adjusting circuit 122b₃ therefore can be changed to adjust and set a value of resonance frequency f_{B}.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

On the other hand, circuit configurations of the inductor 125a that allow the inductance of the entire inductor 125a to be adjusted include, in addition to a single variable inductor, a group of variable inductors in which multiple variable inductors are connected, a group of inductors in which a fixed inductor(s) and a variable inductor(s) are provided in a mixed manner and connected, a group of switching inductors in which fixed inductors are connected with a switch and the switch is turned on and off to change the inductance of the entire inductor circuit, a switch-tapped inductor in which an inductor has multiple taps and switching is made between the taps by using switches to change the inductance, and various other circuit configurations.

Since each of the resonance frequency adjusting circuits 122a₃ and 122b₃ includes a capacitor 124a and an inductor 125a as has been described above in the present embodiment, the values of resonance frequencies f_{A} and f_{B} can be adjusted and set with a high degree of accuracy as in the first embodiment.

### (Seventh embodiment)

An energy supply apparatus according to a seventh embodiment of the present invention will be described below in detail.

The energy supply apparatus of the present embodiment has the same configuration as the energy supply apparatus of the first embodiment described with reference to Fig. 1, including the method in which resonance frequencies are set in power receiving antennas 201a and 201b, in particular the configuration of resonance circuits 203a and 203b contained in power receiving systems 20a and 20b, except the circuit configuration of resonance frequency adjusting circuits 122a₄ and 122b₄. Therefore only the circuit configuration of the resonance frequency adjusting circuits 122a₄ and 122b₄ will be described here and the same components as those of the first embodiment will be given the same reference symbols and description of the same components will be omitted.

A configuration of the resonance frequency adjusting circuits 122a₄ and 122b₄ of the present embodiment will be described with reference to Fig. 8. Fig. 8 is a circuit diagram illustrating a variation of the circuit configuration of the resonance frequency adjusting circuit 122a₄. The resonance frequency adjusting circuit 122a₄ includes a capacitor 124a and an inductor 125a for adjusting and setting reactance. While both of the capacitor 124a and the inductor 125a in the first embodiment are connected to a power transmission coil 123a in series, both of the capacitor 124a and the inductor 125a in the present embodiment are connected to the power transmission coil 123a in parallel.

The value of capacitance of the capacitor 124a and the value of inductance of the inductor 125a are variable and reactance of the entire circuit can be adjusted by adjusting these values. The value of resonance frequency f_{A} can be adjusted and set by changing the reactance of the resonance frequency adjusting circuit 122a₄. The resonance frequency adjusting circuit 122b₄ has the same circuit configuration as the resonance frequency adjusting circuit 122a₄ described with reference to Fig. 8 and therefore detailed description of the resonance frequency adjusting circuit 122b₄ will be omitted. Reactance of the resonance frequency adjusting circuit 122b₄ therefore can be changed to adjust and set a value of resonance frequency f_{B}.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

On the other hand, circuit configurations of the inductor 125a that allow the inductance of the entire inductor 125a to be adjusted include, in addition to a single variable inductor, a group of variable inductors in which multiple variable inductors are connected, a group of inductors in which a fixed inductor(s) and a variable inductor(s) are provided in a mixed manner and connected, a group of switching inductors in which fixed inductors are connected with a switch and the switch is turned on and off to change the inductance of the entire inductor circuit, a switch-tapped inductor in which an inductor has multiple taps and switching is made between the taps by using switches to change the inductance, and various other circuit configurations.

Since each of the resonance frequency adjusting circuits 122a₄ and 122b₄ includes a capacitor 124a and an inductor 125a as described above in the present embodiment, the values of resonance frequencies f_{A} and f_{B} can be adjusted and set with a high degree of accuracy as in the first and sixth embodiments.

### (Eighth embodiment)

An energy supply apparatus according to an eighth embodiment of the present invention will be described below in detail.

The energy supply apparatus of the present embodiment has the same configuration as the energy supply apparatus of the first embodiment described with reference to Fig. 1, including the method in which resonance frequencies are set in power receiving antennas 201a and 201b, in particular the configuration of resonance circuits 203a and 203b contained in power receiving systems 20a and 20b, except the circuit configuration of resonance frequency adjusting circuits 122a₅ and 122b₅. Therefore only the circuit configuration of the resonance frequency adjusting circuits 122a₅ and 122b₅ will be described here and the same components as those of the first embodiment will be given the same reference symbols and description of the same components will be omitted.

A configuration of the resonance frequency adjusting circuits 122a₅ and 122b₅ of the present embodiment will be described with reference to Fig. 9. Fig. 9 is a circuit diagram illustrating a variation of the circuit configuration of the resonance frequency adjusting circuit 122a₅. The resonance frequency adjusting circuit 122a₅ includes a capacitor 124a and an inductor 125a for adjusting and setting reactance. While both of the capacitor 124a and the inductor 125a in the first embodiment are connected to a power transmission coil 123a in series, the capacitor 124a in the present embodiment is connected to the power transmission coil 123a in parallel.

The value of capacitance of the capacitor 124a and the value of inductance of the inductor 125a are variable and reactance of the entire circuit can be adjusted by adjusting these values. The value of resonance frequency f_{A} can be adjusted and set by changing the reactance of the resonance frequency adjusting circuit 122a₅. The resonance frequency adjusting circuit 122b₅ has the same circuit configuration as the resonance frequency adjusting circuit 122a₅ described with reference to Fig. 8 and therefore detailed description of the resonance frequency adjusting circuit 122b₅ will be omitted. Reactance of the resonance frequency adjusting circuit 122b₅ therefore can be changed to adjust and set a value of resonance frequency f_{B}.

Circuit configurations of the capacitor 124a that allow the capacitance of the entire capacitor 124a to be adjusted include, in addition to a single variable-capacitance capacitor, a group of variable-capacitance capacitors in which multiple variable-capacitance capacitors are connected, a group of capacitors in which a fixed-capacitance capacitor(s) and a variable-capacitance capacitor(s) are provided in a mixed manner and connected, a group of switching capacitors in which fixed-capacitance capacitors are connected to a switch and the switch is turned on and off to change the capacitance of the entire capacitor circuit, and various other circuit configurations.

On the other hand, circuit configurations of the inductor 125a that allow the inductance of the entire inductor 125a to be adjusted include, in addition to a single variable inductor, a group of variable inductors in which multiple variable inductors are connected, a group of inductors in which a fixed inductor(s) and a variable inductor(s) are provided in a mixed manner and connected, a group of switching inductors in which fixed inductors are connected with a switch and the switch is turned on and off to change the inductance of the entire inductor circuit, a switch-tapped inductor in which an inductor has multiple taps and switching is made between the taps by using switches to change the inductance, and various other circuit configurations.

Since each of the resonance frequency adjusting circuits 122a₅ and 122b₅ includes a capacitor 124a and an inductor 125a as described above in the present embodiment, the values of resonance frequencies f_{A} and f_{B} can be adjusted and set with a high degree of accuracy as in the first, sixth and seventh embodiments.

According to the embodiments described above, there can be provided an energy supply apparatus capable of stably supplying energy even when multiple such energy supply apparatuses are located close to each other.

While a small medical device included in an energy supply apparatus of the present invention has been described with respect to a small medical apparatus including an image pickup unit, or what is called a capsule endoscope, by way of example in the eight embodiments, the present invention is not limited to the embodiments described above. Various changes and modifications can be made to the embodiments without departing from the spirit of the present invention.

For example, the present invention is also applicable to an ingestible pH measuring device, which is swallowed by a subject and measures pH in the body of the subject, and an ingestible thermometer, which is swallowed by a subject and measures internal body temperature.

### Industrial Applicability

It will be understood that the power transmission system of the present invention is applicable to a wide variety of apparatuses that wirelessly supply electric power, in addition to small medical devices mentioned above.

This application is based upon and claims the benefit of priority from the prior Japanese Patent Application No. 2008-111608 filed in Japan on April 22, 2008, the entire contents disclosed in which are incorporated herein and the claims by reference.

## Claims

1. A power transmission system for supplying energy to a device operating on electrical energy taken by a power receiving antenna, the power transmission system comprising:
a driving unit supplied with electric power from a power supply and generating an AC current;
a power transmission antenna receiving the AC current from the driving unit and generating an electromagnetic field to supply the electrical energy to the device; and
a resonance frequency adjusting circuit provided in the power transmission antenna and adjusting and setting a resonance frequency.

2. The power transmission system according to claim 1, wherein the resonance frequency adjusting circuit adjusts and sets the resonance frequency of the power transmission antenna to a frequency different from a resonance frequency of a power transmission antenna provided in another of the energy supply apparatus located close to the energy supply apparatus.

3. The power transmission system according to claim 1, wherein the resonance frequency adjusting circuit adjusts and sets the resonance frequency of the power transmission antenna to a frequency that reduces an induced current induced in a power transmission antenna provided in another of the energy supply apparatus located close to the energy supply apparatus.

4. The power transmission system according to any one of claims 1 to 3, wherein reactance of the resonance frequency adjusting circuit is adjustable.

5. The power transmission system according to any one of claims 1 to 4, wherein the resonance frequency adjusting circuit comprises a circuit including one or more capacitors and/or one or more inductors and a capacitance of at least one capacitor or inductance of at least one inductor is variable.

6. The power transmission system according to any one of claims 1 to 5, wherein the resonance frequency of the power receiving antenna is equal to a resonance frequency of the power transmission antenna.

7. The power transmission system according to any one of claims 1 to 5, wherein the power receiving antenna comprises a resonance circuit adjusting and setting a resonance frequency and reactance of the resonance circuit is adjustable.

8. The power transmission system according to any one of claims 1 to 5, wherein the resonance frequency of the power receiving antenna has a frequency characteristic that enables minimum electric power required for causing the device to operate to be taken in a frequency band from a minimum value to a maximum value of the resonance frequency that can be set in the power transmission antenna.

9. The power transmission system according to claim 6, wherein the resonance frequency of the power receiving antenna is fixed.

10. The power transmission system according to claim 8, wherein the resonance frequency of the power receiving antenna is fixed.

11. The power transmission system according to claim 5, wherein the at least one capacitor is connected to a power transmission coil of the power transmission antenna in series.

12. The power transmission system according to claim 5, wherein the at least one capacitor is connected to a power transmission coil of the power transmission antenna in parallel.

13. The power transmission system according to claim 5, wherein the at least one capacitor is connected to a power transmission coil of the power transmission antenna in series and the at least one inductor is connected to the power transmission coil of the power transmission antenna in parallel.

14. The power transmission system according to claim 5, wherein the at least one capacitor and the at least one inductor are connected to the power transmission coil of the power transmission antenna in parallel.

15. The power transmission system according to claim 5, wherein the at least one capacitor is connected to a power transmission coil of the power transmission antenna in parallel and the at least one inductor is connected to the power transmission coil of the power transmission antenna in series.
